# EUROPEAN PATENT APPLICATION

(11) **EP 1 616 591 A2**
(43) Date of publication of application: **18.01.2006**
(21) Application number: 05254159.6
(22) Date of filing: 01.07.2005
(51) Int. Cl.: A61M 5/315

(54) **Syringe with separate cavities**

(30) Priority: 12.07.2004 US 890330
(71) Applicant: Tai, Cheh, Zhongli City T'ao yuan 320 (TW)
(72) Inventor: Tai, Cheh, Zhongli City T'ao yuan 320 (TW)
(74) Representative: Mounteney, Simon James

(57) **Abstract**

The present invention discloses a syringe (100) with separate cavities (140A,140B). It comprises an empty body and a plunger assembly (170). The plunger assembly can be plug-in the empty body. Two separate cavities, a first cavity and a second cavity, are roomed at specified positions inside the empty body. And a drain is used to connect these two separate cavities. The plunger assembly comprises a straight elongated stem, a piston head, and at least two fastening elements.

## Description

### BACKGROUND OF THE INVENTION

### 1. FIELD OF THE INVENTION

The present invention generally relates to syringe, and more particularly to syringe with separate cavities.

### 2. DESCRIPTION OF THE PRIOR ART

In many medical environments, it is necessary that a drug is injected into a patient during diagnosis or treatment. Medical syringes are used in both injection and aspiration modes. Conventionally, when a syringe is used to inject medicine via a needle into a venous or the like, the operator holds the syringe in one hand and squeezes the plunger into the syringe cylinder using the thumb and fingers of the same hand. At the present time there are three basic types of injections given with a hypodermic apparatus. One type involves the injection of the hypodermic into the fatty area or fatty tissues of the body, and is commonly referred to as a "Sub-Q" injection. Another type is the application of the hypodermic apparatus directly into a venous, and this is commonly known as an "IV" or an intravenous injection. This third of the basic types of hypodermic usage is the application of the apparatus directly into a muscular area, and this is usually referred to as an "IM" or intramuscular injection. In general, the drug includes fluid and powder. Certain medicines which must be administered to patients by injection consist of lyophilized powder dissolved in a diluent. The powder and diluent must be mixed together immediately before use, since if the powder and diluent are mixed at an earlier time, the medicine will deteriorate rapidly. Conventionally, the powder and diluent are mixed at time of dosage and the medicine is transferred into a syringe for immediate subsequent injection.

### SUMMARY OF THE INVENTION

According to the description above, the present invention provides a syringe with separate cavities to meet the convenience requirements of users in addition to economic benefits of industry.

One objective of the present invention is to provide a syringe with separate cavities to beforehand store separate medicinal components which can be mixed right at dosage. The syringe comprises at least one drain and two separating elements to room at least two separate cavities inside an empty body of the syringe. In this regards, the drain is used to connect these two separate cavities such that the diluent can mix up with the powder through the drain at dosage. In addition, the present invention also provides a fastening mechanism to prevent the plunger assembly being dragged by the attraction force caused by the negative pressure inside these separate cavities. The drag will lead to the mix up with the powder and the diluent and the leakage of medicine. Hence, the present invention can achieve the objective to store separate medicinal components in one syringe. It helps to fulfill the desired objective and the requirements of medicinal industry.

In accordance with the objectives mentioned above, the present invention discloses a syringe with separate cavities which comprises an empty body and a plunger assembly. The empty space inside the body is bounded by a first sidewall with a diameter and a second sidewall with a first caliber. A first end of the body is an open end. The caliber of the open end is as the same as the first caliber. In addition, a second end of the body is stretched as a nozzle wherein the caliber of the nozzle designated as a second caliber is smaller than the first caliber. A first separate cavity and a second separate cavity are roomed at some specified parts of the empty body. In this regards, the second separate cavity is connected to the nozzle and comprises at least a drain. The plunger assembly comprises a straight elongated stem, at least two fastening elements, and at least two piston heads wherein the caliber of these two piston heads is as the same as the first caliber. In this regards, a first end of the straight elongated stem is a handle of the plunger assembly. A second end of the straight elongated stem is connected to the first piston head which is closer to the open end. Therefore, the first and second piston heads are considered as separating elements to room the first and a second separate cavities inside an empty body of the syringe. On the other hand, at least two fastening elements are provided at the opposite position on the surface of the straight elongated stem to fix it to the empty body.

### BRIEF DESCRIPTION OF THE DRAWINGS

The accompanying drawings incorporated in and forming a part of the specification illustrate several aspects of the present invention, and together with the description serve to explain the principles of the invention. In the drawings:
FIG. 1A to 1F shows mechanical profile diagrams illustrating the syringe with separate cavities provided by a first embodiment of the present invention;
FIG. 1G to 1I depicts diagrams illustrating a practical operation of the syringe with separate cavities provided by the first embodiment of the present invention;
FIG. 2A to 2E shows mechanical profile diagrams illustrating the syringe with separate cavities provided by an second embodiment of the present invention;
FIG. 2F to 2H depicts diagrams illustrating a practical operation of the syringe with separate cavities provided by the second embodiment of the present invention;
FIG. 3A to 3D shows mechanical profile diagrams illustrating the syringe with separate cavities provided by an third embodiment of the present invention; and
FIG. 3E to 3G depicts diagrams illustrating a practical operation of the syringe with separate cavities provided by the third embodiment of the present invention.

### DESCRIPTION OF THE PREFERRED EMBODIMENT(S)

Having summarized various aspects of the present invention, reference will now be made in detail to the description of embodiments of the invention as illustrated in the drawings. While embodiments of the invention will be described in connection with these drawings, there is no intent to limit it to the embodiment or embodiments disclosed therein. On the contrary the intent is to cover all alternatives, modifications and equivalents included within the spirit and scope of the invention as defined by the appended claims.

It is noted that the drawings presents herein have been provided to illustrate certain features and aspects of embodiments of the invention. It will be appreciated from the description provided herein that a variety of alternative embodiments and implementations may be realized, consistent with the scope and spirit of the present invention.

It is also noted that the drawings presents herein are not consistent with the same scale. Some scales of some components are not proportional to the scales of other components in order to provide comprehensive descriptions and emphasizes to embodiments of this present invention.

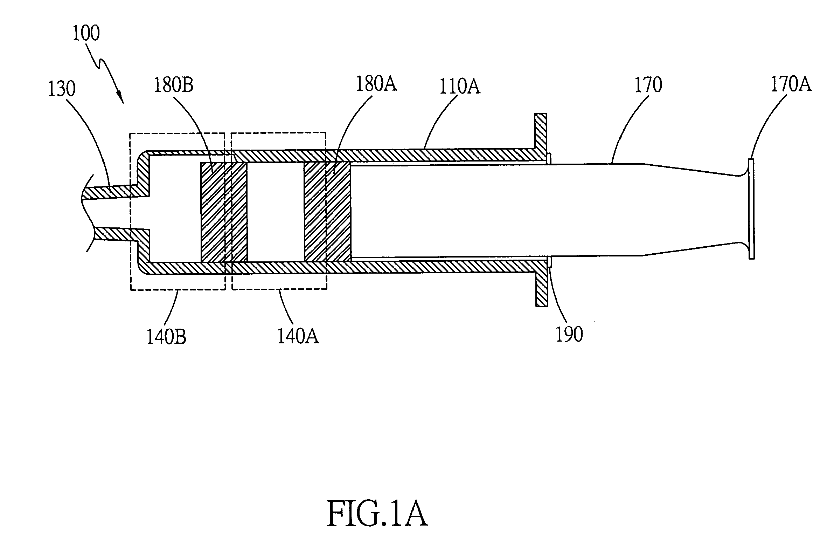

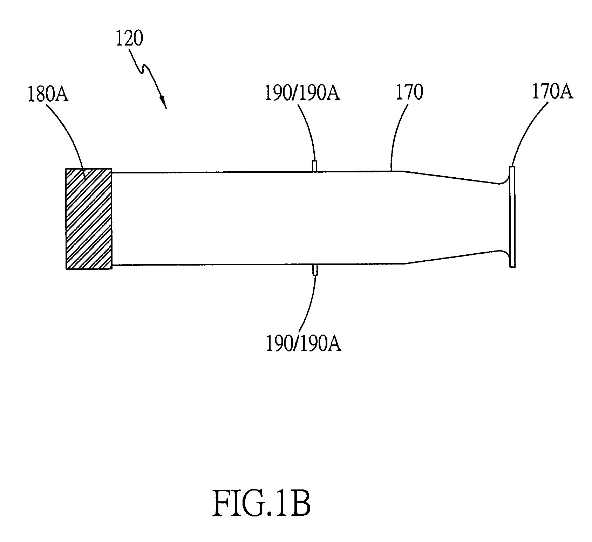

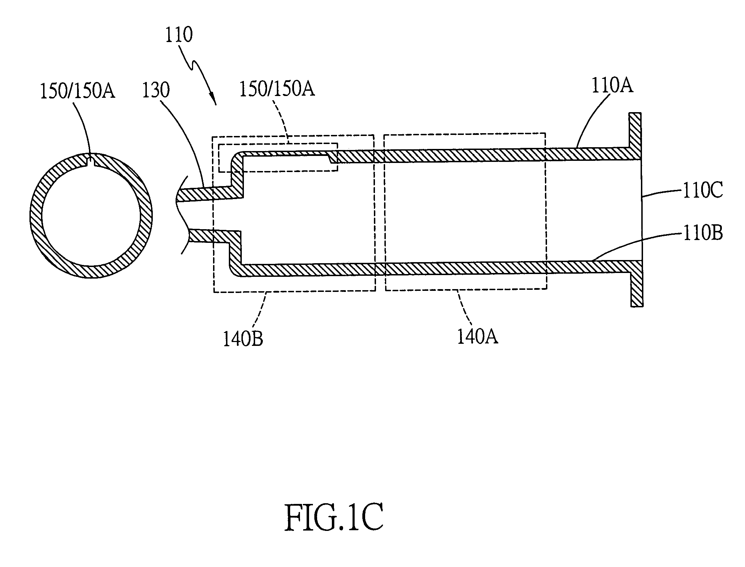

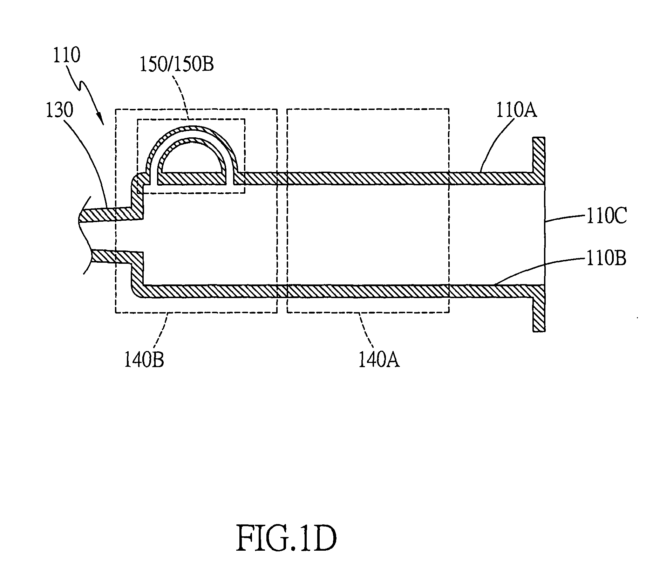

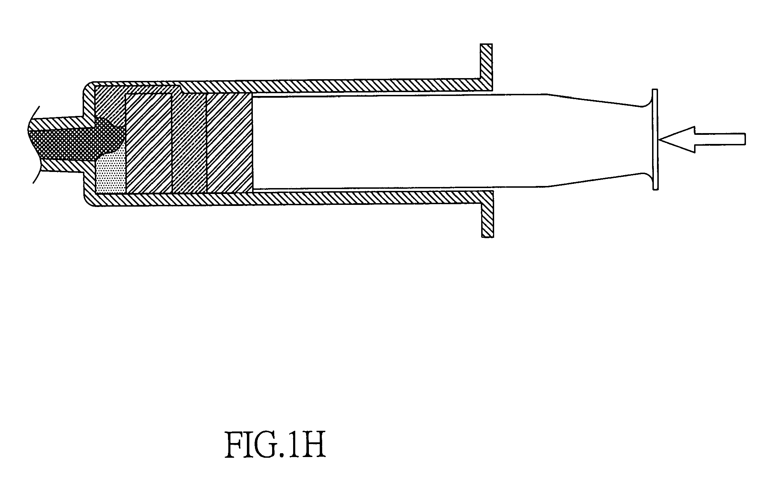

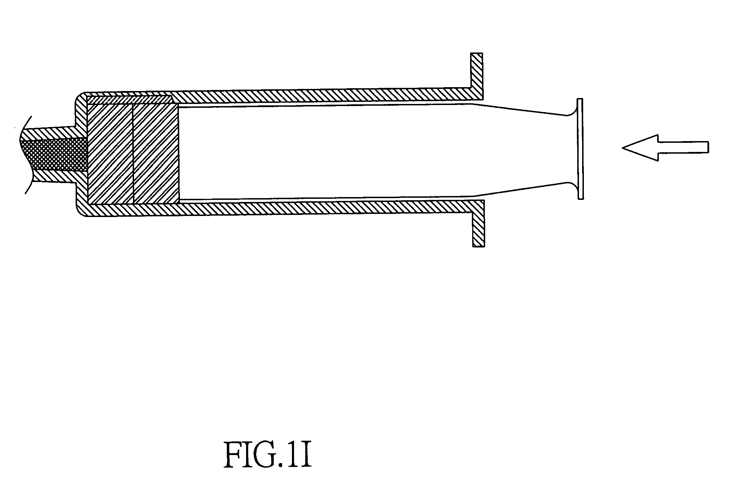

Referring to FIG. 1A to 1D, in accordance with a first preferred embodiment of the present invention, a provided syringe 100 with separate cavities comprises an empty body 110 and a plunger assembly 120. In this regards, the plunger assembly 120 can be plug-in the empty space inside the body 110. The empty space inside the body 110 is bounded by a first sidewall 110A with a diameter and a second sidewall 110B with a first caliber. A first end of the body 110 is an open end 110C. The caliber of the open end 110C is as the same as the first caliber. In addition, a second end of the body 110 is stretched as a nozzle 130 wherein the caliber of the nozzle 130 designated as a second caliber is smaller than the first caliber. According to the embodiment of the present invention, the plunger assembly 120 comprises a straight elongated stem 170, at least two fastening elements 190, and at least two piston heads 180A/180B wherein the caliber of these two piston heads 180A/180B is as the same as the first caliber. In this regards, a first end of the straight elongated stem 170 is a handle 170A of the plunger assembly 120. A second end of the straight elongated stem 170 is connected to the first piston head 180A which is closer to the open end 110C than the nozzle 130. Moreover, the second piston head 180B is located at a certain distance from the first piston head 180A such that the empty space of the body 110 is divided into two separate empty cavities, a first separate cavity 140A and a second separate cavity 140B. In this regards, the second separate cavity 140B is connected to the nozzle 130 and comprises at least a drain 150. The drain 150 can be a conduit 150A at the surface of the second sidewall 110B which is surrounding the second separate cavity 140B of the empty body 110. The depth of the conduit 150A is less than the difference between the diameter and the first caliber. Besides, the drain 150 can be a conduit 150B for fluid flow, too. Both ends of the conduit 150B are opened at the surface of the second sidewall 110B to communicate the second separate cavity 140B and the first sidewall 110A is penetrated by the conduit 150B.

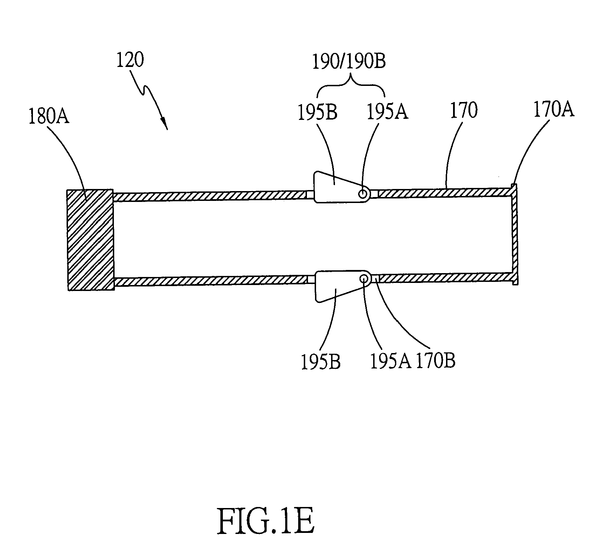

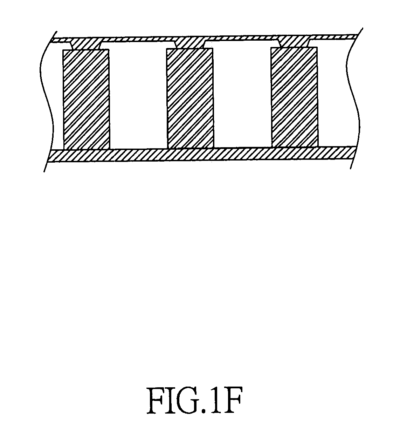

As shown in FIG. 1A to 1D, in accordance with the embodiment of the present invention, the thickness of the second piston head 180B is slightly less than the length of the conduit 150A and/or the distance between the both ends of the conduit 150B. However, the total thickness of these two piston heads 180A/ 180B is larger than the length of the conduit 150A and/or the distance between the both ends of the conduit 150B. As shown in FIG. 1E, these two fastening elements 190/ 190A are at the opposite positions on the sidewall of the straight elongated stem 170 such that the straight elongated stem 170 could be fixed to the open end 110C of the body 110 in order to prevent these two piston heads dragging by the negative pressure inside these separate cavities. Furthermore, the fastening element 190/190B comprises an elastic element 195A and a flanker wing 195B inside the inner space within the straight elongated stem 170. Two open holes 170B are placed at the opposite positions along the straight elongated stem 170 such that the flanker wings 195B can swing in and out of these two open holes 170B freely. In addition, these fastening elements 190/ 190B can be fastening structures at the surfaces of the straight elongated stem 170. Whenever the straight elongated stem 170 is pushed, these fastening structures will be broken at the moment. The present invention also adapts to a syringe with a plurality of separate cavities which are roomed by a plurality of piston heads. The number of the plurality of separate cavities equals to the number of the plurality of piston heads. Besides, each of the plurality of separate cavities comprises at least one drain as shown in FIG. 1F.

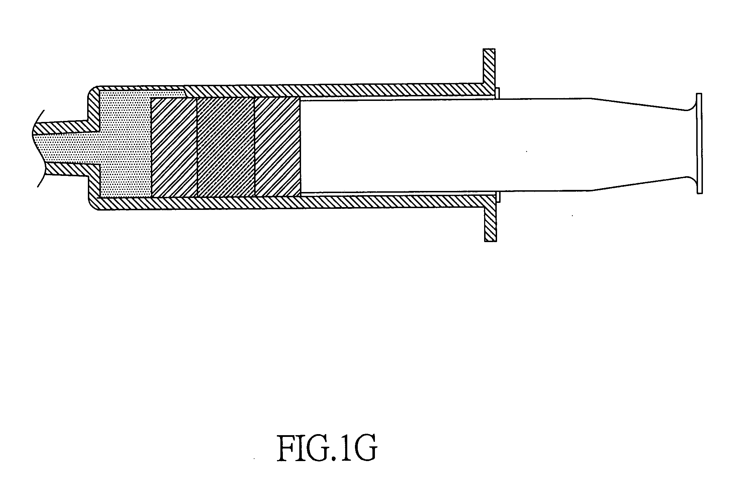

Referring to FIG 1G and 1I, a practice example of the syringe with separate cavities 100 is shown in accordance with the embodiment of the present invention. At first, medicinal powder is placed into the empty space of the body 110. Following, the second piston head 180B and the plunger assembly 120 are put orderly inside the open end 110C of the body 110. The handle 170A of the plunger assembly 120 is pushed until the second piston head 180B reaching a proper position such that the first separate cavity 140A and the second separate cavity 140B are roomed properly. Next, the straight elongated stem 170 of the plunger assembly 120 is taken out and the second piston head 180B is remained inside the empty space of the body 110. Therefore the second separate cavity 140B is formed and filled of the medicinal powder. The fluid is placed in the remained empty space other than the second separate cavity 140B of the body 110 in the next step. At last, the handler 170A of the plunger assembly 120 is pushed until the first separate cavity 140A is enclosed and filled of the fluid such that the plunger assembly 120 can be fixed by these fastening elements 190 of the straight elongated stem 170 at the open end 110C.

When the syringe with separate cavities 100 is ready to use, these two fastening elements 190/ 190A are broken or the two fastening elements 190/ 190B are swung inside the straight elongated stem 170 at first such that the handle 170A of the plunger assembly 120 can be pushed inward. Due to the attraction force from the negative pressure of the first separate cavity 140A and the second separate cavity 140B, the second piston head 180B is pushed inward to compress the second separate cavity 140B by the advance of the plunger assembly 120. When the advance of the second piston head 180B exposes a portion of the conduit 150A or one open end of the conduit 150B, the pressure of these two connected separate cavities is balanced and the second piston head 180B is ceased to move. At this moment, the advance of the first piston head 180A remained and starts to compress the space of the first separate cavity 140A such that the fluid in the first separate cavity 140A is drained to mix the powder in the second separate cavity 140B through the drain 150. When the space of the first separate cavity 140A is completely compressed by the first piston head 180A, the second separate cavity 140B is compressed by the advance of the second piston head 180B which is also pushed by the advance of the first piston head 180A. Therefore the mixed medicine is injected from the nozzle 130 of the body 110. When the space of the second cavity 140B is completely compressed out, all mixed medicine is pushed out of the body 110.

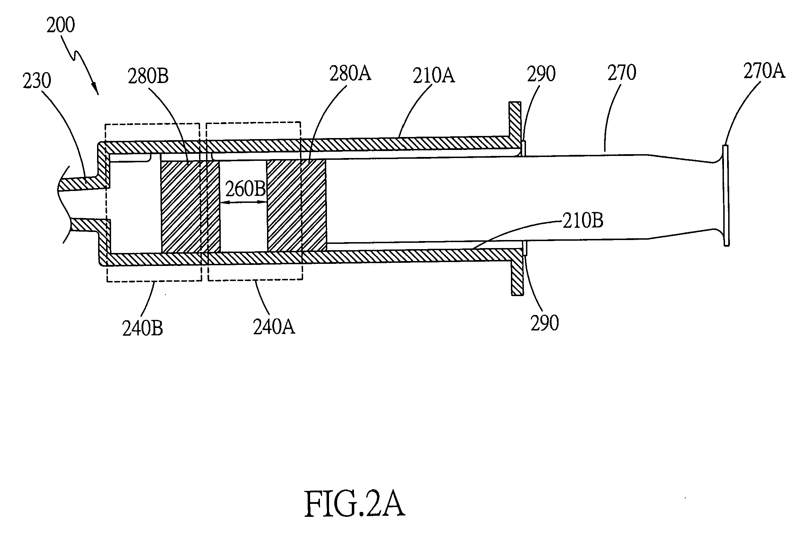

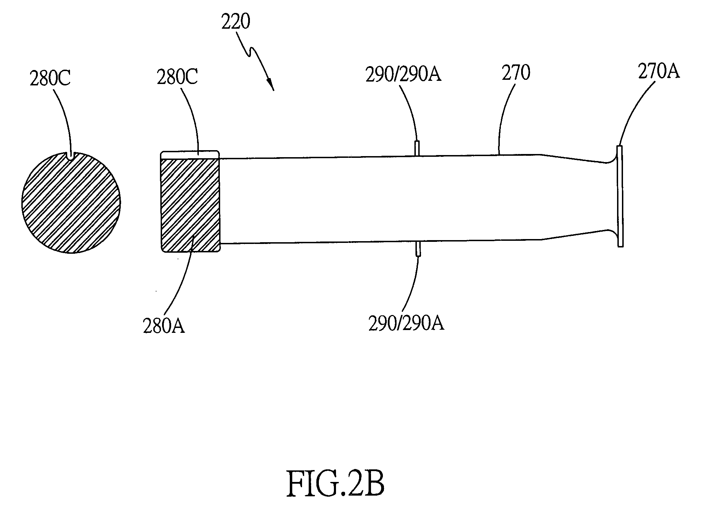

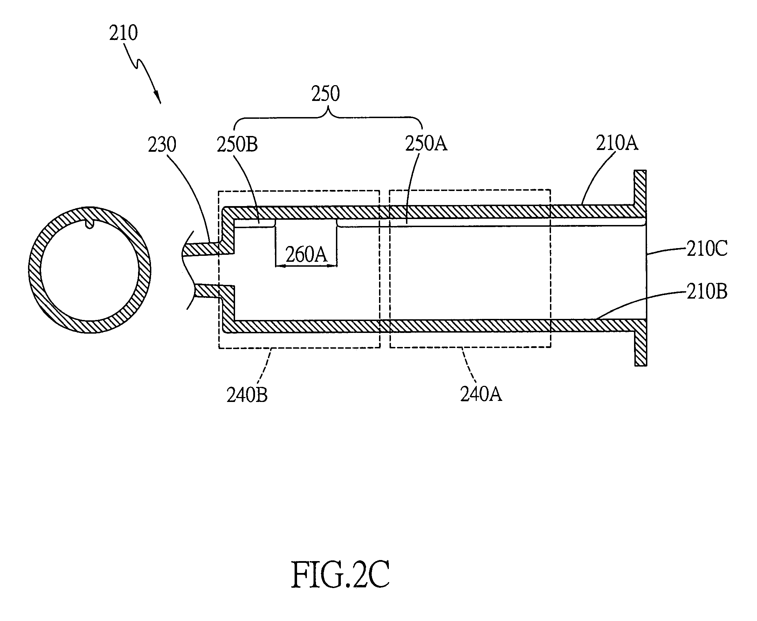

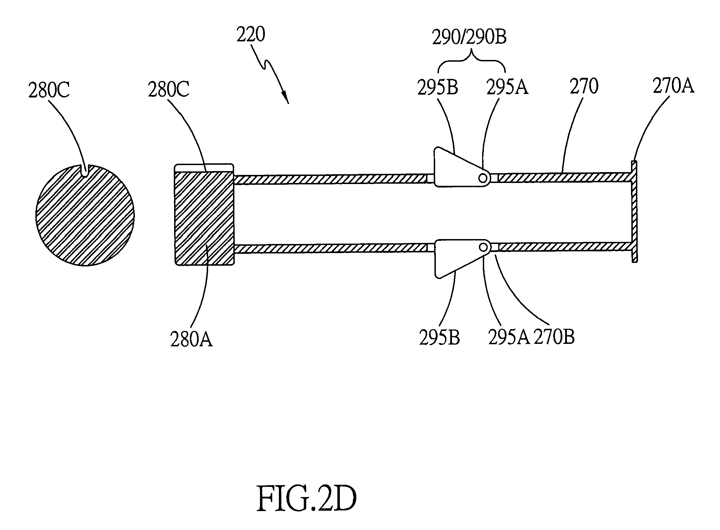

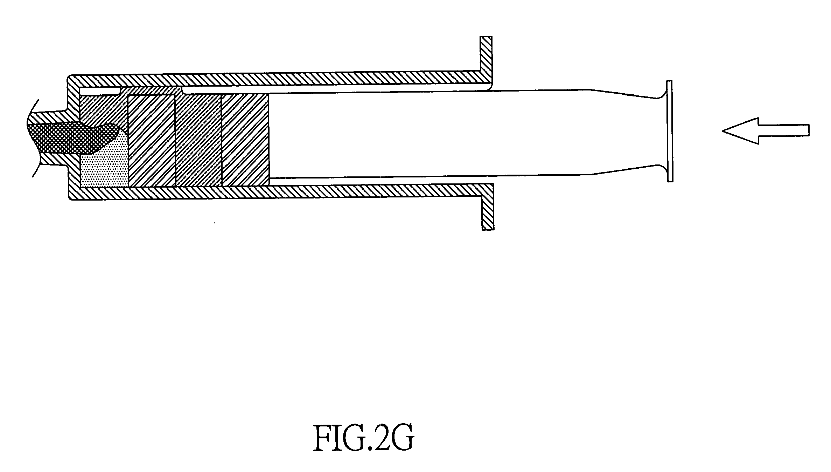

Referring to FIG 2A to 2D, a provided syringe 200 with separate cavities is provided according to a second preferred embodiment of the present invention. The syringe 200 with separate cavities comprises an empty body 210 and a plunger assembly 220. In this regards, the plunger assembly 220 can be plug-in the empty space inside the body 210. The empty body 210 is constructed by a first sidewall 210A with a diameter and a second sidewall 210B wherein the caliber of the second sidewall 210B is designated as the first caliber. A first end of the body 210 is an open end 210C. The caliber of the open end 210C is as the same as the first caliber. In addition, a second end of the body 210 is stretched as a nozzle 230 wherein the caliber of the nozzle 230 designated as the second caliber is smaller than the first caliber. At least a leading guide 250 is located at the surface of the second sidewall 210B. Each leading guide 250 comprises a first protrusion bar 250A and a second protrusion bar 250B. These two protrusion bars 250A and 250B are line up and separated in a first distance 260A.

As shown in FIG 2A to 2D, according to the embodiment of the present invention, the plunger assembly 220 comprises a straight elongated stem 270, at least two fastening elements 290, and at least two piston heads 280A/280B wherein the caliber of these twp piston heads 280A/280B is as the same as the first caliber. In this regards, a first end of the straight elongated stem 270 is a handle 270A of the plunger assembly 220. A second end of the straight elongated stem 270 is connected to the first piston head 280A which is closer to the open end 210C than the nozzle 230. There is a flute 280C on the surface of the first piston head 280A and the second piston head 280B to fit the first protrusion bar 250A and the second protrusion bar 250B. Moreover, the second piston head 280B is located at a second distance 260B from the first piston head 280A such that the empty space of the body 210 is divided into two separate empty cavities, a first separate cavity 240A and a second separate cavity 240B. Besides, the length of the first separate cavity 240A is the second distance 260B.

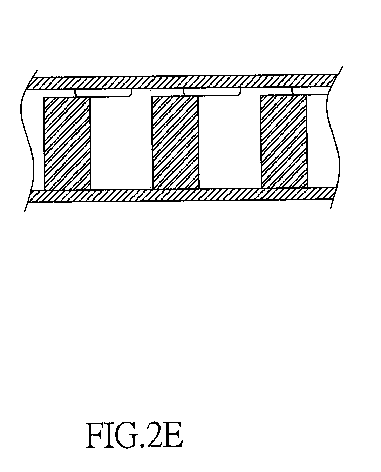

As shown in FIG 2A to 2D, the second separate cavity 240B is connected to the nozzle 230 with the second caliber according to the embodiment of the present invention. The second cavity 240B occupies some part of the empty body 210. And the second cavity 240B is some part of empty space of the body which comprises a portion of the first protrusion bar 250A, the first distance 260A, and a portion of the second protrusion bar 250B. The thickness of the second piston head 280B is slightly less than the first distance 260A and the total thickness of the first piston head 180A and the second piston head 180B is longer than the first distance 260A. The first separate cavity 240A occupies some empty space of the body 210 which includes a portion of the first protrusion bar 250A. These two fastening elements 290/290A are at the opposite positions on the sidewall of the straight elongated stem 270 such that the straight elongated stem 270 could be fixed to the open end 210C of the body 210 in order to prevent these two piston heads 280A/280B dragging by the negative pressure inside these two separate cavities. In addition, at least two fastening elements 290/290B are located at two open holes 270B at the opposite positions of the straight elongated stem 270. Furthermore, the fastening element 290/290B comprises an elastic element 295A and a flanker wing 295B. The flanker wings 295B can swing in and out of these two open holes 270B freely. Besides, as shown in FIG. 2E, the present invention also adapts to a syringe with a plurality of separate cavities which are roomed by a plurality of piston heads. The number of the plurality of separate cavities equals to the number of the plurality of piston heads. Each of the separate cavities comprises at least a second protrusion. And every second protrusion is separated in the first distance.

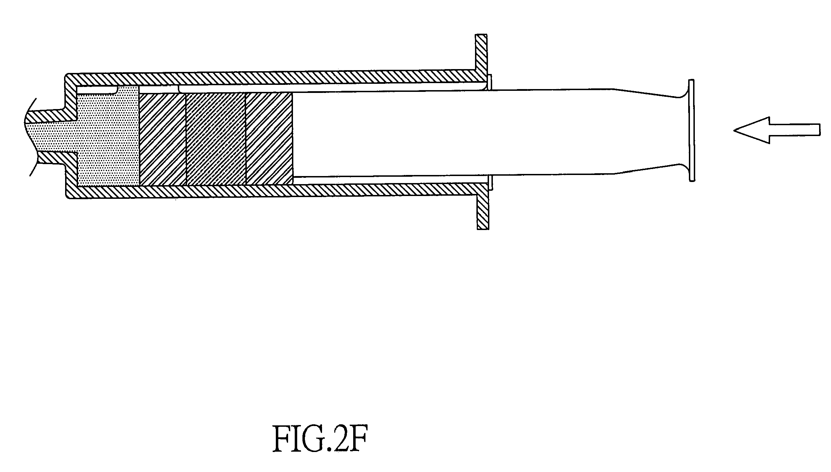

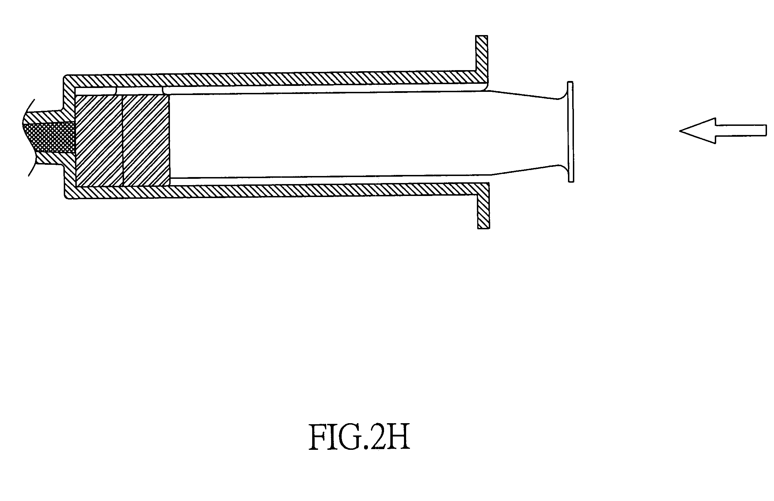

Referring to FIG 2F and 2H, a practice example of the syringe with separate cavities 200 is shown in accordance with the embodiment of the present invention. At first, medicinal powder is put into the empty space of the body 210. Following, the second piston head 280B is put inside the open end 210C of the body 210 by matching the flute 280C with the first protrusion bar 250A of the second sidewall 210B. The handle 270A of the plunger assembly 220 is pushed until the second piston head 280B reaching a proper position such that the second separate cavity 240B is roomed. Next, the straight elongated stem 270 of the plunger assembly 220 is taken out and the second piston head 280B is remained inside the empty space of the body 210. Therefore the second separate cavity 240B is filled of the medicinal powder. The fluid is placed in the remained empty space of the body 210 in the next step. At last, the handler 270A of the plunger assembly 120 is pushed along the direction of the first protrusion bar 250A until the fastening elements 290A of the straight elongated stem 270 can fix the plunger assembly at the open end 210C such that the first separate cavity 240A is enclosed and filled of the fluid and the second separate cavity 240B is filled of the medicinal powder.

When the syringe with separate cavities 200 is ready to use, these two fastening elements 290/290A are broken or the two fastening elements 290/290B are swung inside the straight elongated stem 270 such that the handle 270A of the plunger assembly 220 can be pushed inward. Due to the attraction force from the negative pressure of the first separate cavity 240A and the second separate cavity 240B, the second piston head 280B is pushed inward to compress the second separate cavity 240B by the advance of the plunger assembly 220. When the advance of the second piston head 280B is completely relief the first protrusion bar 250A and entered the empty space within the first distance 260A between the first protrusion bar 250A and the second protrusion bar 250B, the pressure of these two connected separate cavities is balanced and the second piston head 280B is ceased to move. At this moment, the advance of the first piston head 280A starts to compress the space of the first separate cavity 240A along the direction of the first protrusion bar 250A such that the fluid in the first separate cavity 240A is drained to mix the powder in the second separate cavity 240B through the flute 280C of the second piston head 280B. When the space of the first separate cavity 240A is completely out by the advance of the first piston head 280A, the second separate cavity 240B starts to be compressed by the advance of the second piston head 280B which is also pushed by the advance of the first piston head 280A. Therefore the mixed medicine is injected from the nozzle 230 out of the body 210. Besides, the flute 280C of the first piston head 280A and the second piston head 280B is matched with the second protrusion bar 250B. When the space of the second cavity 240B is completely compressed out, all mixed medicine is pushed out of the body 210.

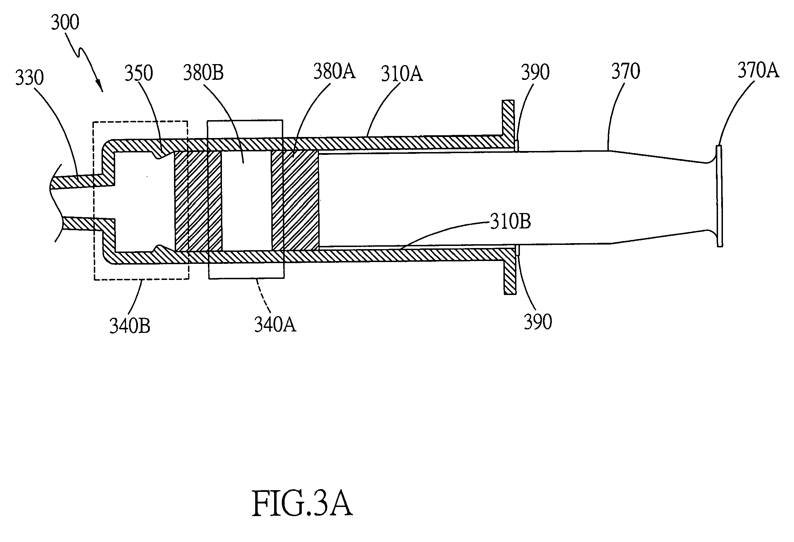

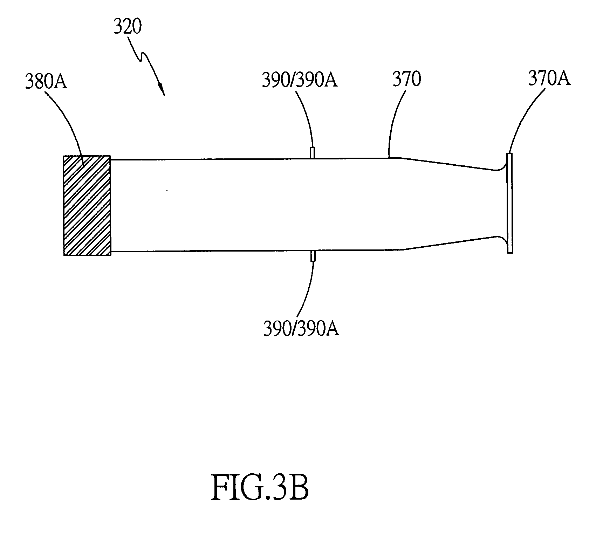

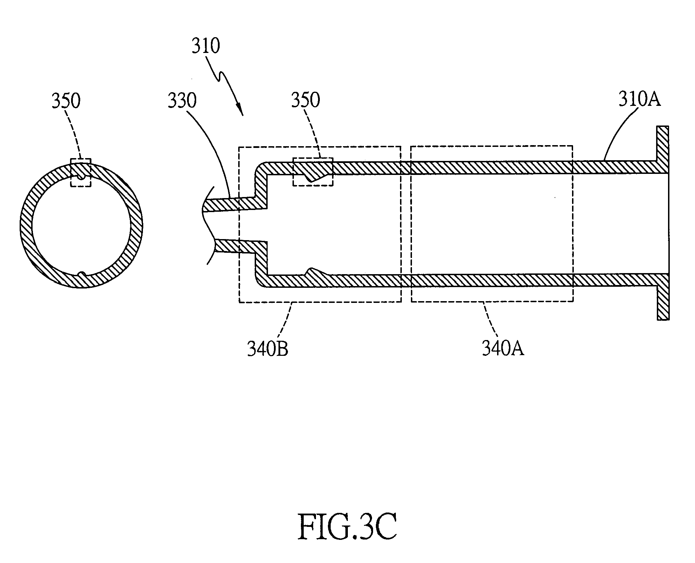

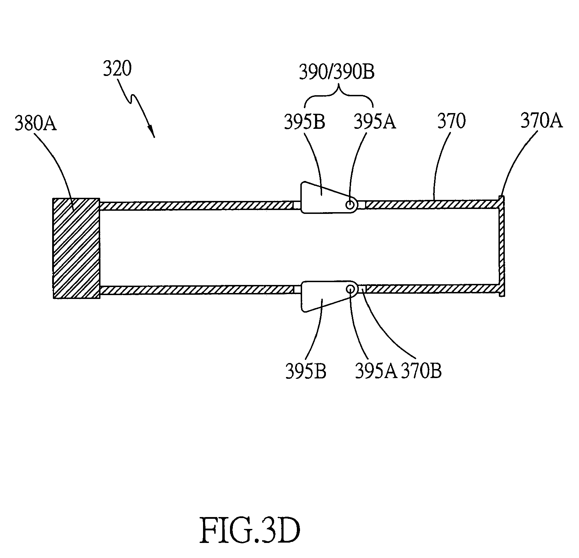

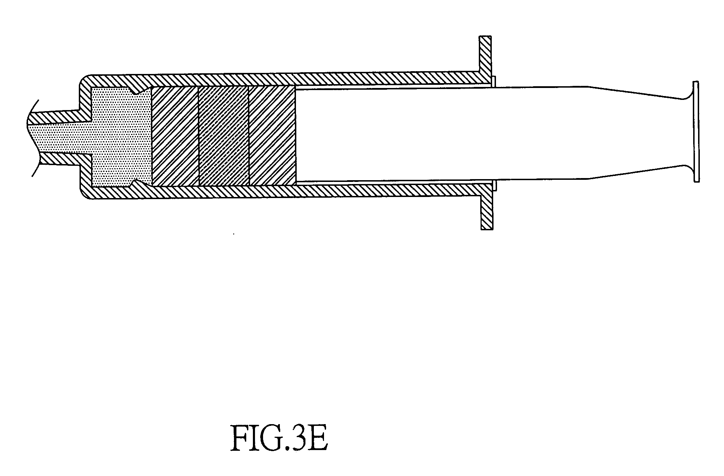

Referring to FIG 3A to 3D, a provided syringe 300 with separate cavities is provided according to a third preferred embodiment of the present invention. The syringe 300 with separate cavities comprises an empty body 310 and a plunger assembly 320. In this regards, the plunger assembly 320 can be plug-in the empty space inside the body 310. The empty body 310 is constructed by a first sidewall 310A with a diameter and a second sidewall 310B wherein the caliber of the second sidewall 310B is designated as the first caliber. A first end of the body 310 is an open end 310C. The caliber of the open end 310C is as the same as the first caliber. In addition, a second end of the body 310 is stretched as a nozzle 330 wherein the caliber of the nozzle 330 designated as the second caliber is smaller than the first caliber. At least a protrusion 350 is located at the surface of the second sidewall 310B.

As shown in FIG 3A to 3D, according to the embodiment of the present invention, the plunger assembly 320 comprises a straight elongated stem 370, at least two fastening elements 390, and at least two piston heads 380A/380B wherein the caliber of these two piston heads 380A/ 380B is as the same as the first caliber. In this regards, a first end of the straight elongated stem 370 is a handle 370A of the plunger assembly 320. A second end of the straight elongated stem 370 is connected to the first piston head 380A which is closer to the open end 310C than the nozzle 330. Moreover, the second piston head 380B is located at a distance from the first piston head 380A such that the empty space of the body 310 is divided into two separate empty cavities, a first separate cavity 340A and a second separate cavity 340B.

As shown in FIG 3A to 3D, the second separate cavity 340B is connected to the nozzle 330 with the second caliber according to the embodiment of the present invention. The second cavity 340B occupies some part of the empty body 310. And the second cavity 340B is as long as the protrusion 350. These two fastening elements 390/390A are at the opposite positions on the sidewall of the straight elongated stem 370 such that the straight elongated stem 370 could be fixed to the open end 310C of the body 310 in order to prevent these two piston heads 380A/ 380B dragging by the negative pressure inside these two separate cavities. In addition, at least two fastening elements 390/390B are located at two open holes 370B at the opposite positions of the straight elongated stem 370. Furthermore, the fastening element 390/390B comprises an elastic element 395A and a flanker wing 395B. The flanker wings 395B can swing in and out of these two open holes 370B freely.

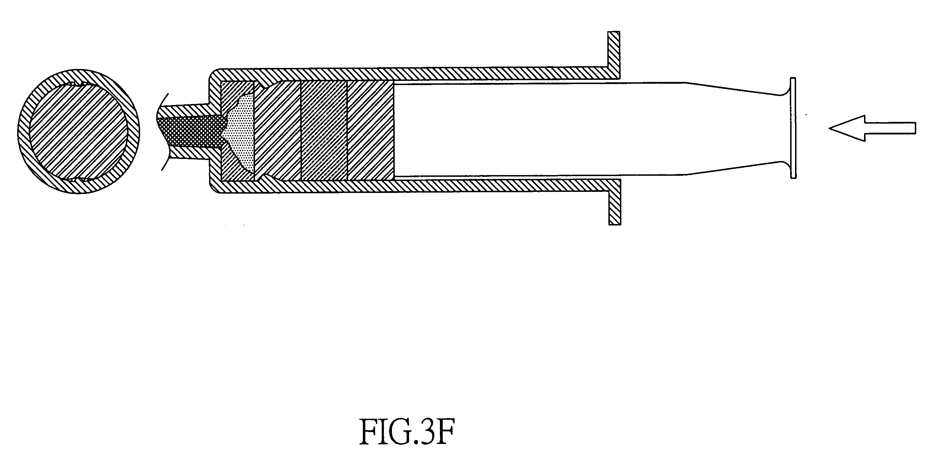

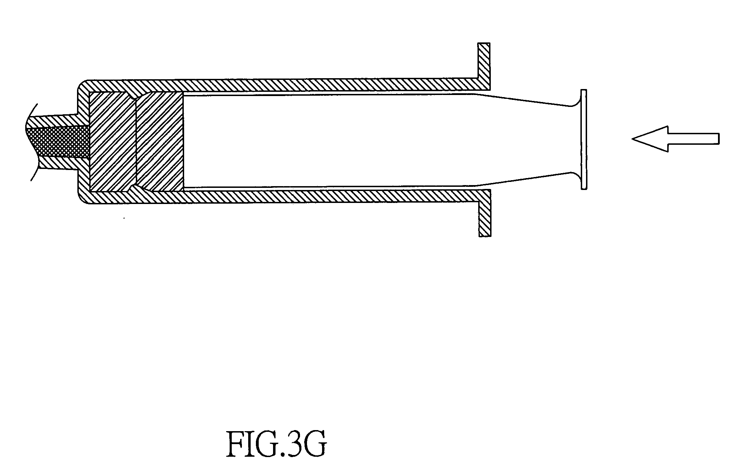

Referring to FIG 3F and 3G, a practice example of the syringe with separate cavities 300 is shown in accordance with the embodiment of the present invention. At first, medicinal powder is put into the empty space of the body 310. Following, the second piston head 380B is put inside the open end 310C of the body 310. The handle 370A of the plunger assembly 320 is pushed until the second piston head 380B reaching a proper position. Next, the straight elongated stem 370 of the plunger assembly 320 is taken out and the second piston head 380B is remained inside the empty space of the body 310. Therefore the second separate cavity 340B is filled of the medicinal powder. The fluid is placed in the remained empty space of the body 310 in the next step. At last, the handler 370A of the plunger assembly 320 is pushed until the fastening elements 390 of the straight elongated stem 370 can fix the plunger assembly at the open end 310C such that the first separate cavity 340A is enclosed and filled of the fluid and the second separate cavity 340B is filled of the medicinal powder. The protrusion 350 is within the second separate cavity 340B.

When the syringe with separate cavities 300 is ready to use, these two fastening elements 390/390A are broken or the two fastening elements 390/390B are swung inside the straight elongated stem 370 such that the handle 370A of the plunger assembly 320 can be pushed inward. Due to the attraction force from the negative pressure of the first separate cavity 340A and the second separate cavity 340B, the second piston head 380B is pushed inward to compress the second separate cavity 340B by the advance of the plunger assembly 320. When the advance of the second piston head 380B is reached to the protrusion 350, the second piston head 380B will be deformed by the squeezing effect of the protrusion 350. A transient drain between these two connected separate cavities is produced by the squeezing process. Hence the pressure of these two connected separate cavities is balanced and the second piston head 380B is ceased to move. At this moment, the advance of the first piston head 380A starts to compress the space of the first separate cavity 340A such that the fluid in the first separate cavity 340A is drained to mix the powder in the second separate cavity 340B through the transient formed by the squeeze of the protrusion 350. When the space of the first separate cavity 340A is completely out by the advance of the first piston head 380A, the second separate cavity 340B starts to be compressed by the advance of the second piston head 380B which is also pushed by the advance of the first piston head 380A. Therefore the mixed medicine is injected from the nozzle 330 out of the body 310. Besides, the second piston head 380B and the first piston head 380A will orderly get out of the squeeze the protrusion 350, respectively. When the space of the second cavity 340B is completely compressed out, all mixed medicine is pushed out of the body 310.

In addition to these mentioned embodiments, an important concept provided by the present invention is that the number of the plurality of separate cavities equals to the number of the plurality of piston heads. Besides, a drain is provided to each of the plurality of separate cavities. The present invention does not only apply to the medicinal syringe, but also to any injection apparatus in biochemical and chemical applications. In addition, the syringe with separate cavities provided by the present invention is not applied in such related fields yet.

The foregoing description is not intended to be exhaustive or to limit the invention to the precise forms disclosed. Obvious modifications or variations are possible in light of the above teachings. In this regard, the embodiment or embodiments discussed were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the inventions as determined by the appended claims when interpreted in accordance with the breath to which they are fairly and legally entitled.

## Claims

1. A syringe with separate cavities, comprising:
an empty body wherein a first end of said body is an open end with a first caliber, a second end of said body is stretched as a nozzle with a second caliber which is smaller than said first caliber, the empty space inside said body is bounded by a first sidewall with a diameter and a second sidewall with said first caliber;
an plunger assembly which can be plug-in said empty body wherein said plunger assembly further comprises a straight elongated stem and a plurality of piston heads with said first caliber, a first end of said straight elongated stem is a handle, a second end of said straight elongated stem is connected to a first piston head of said plurality of piston heads, the rest of said plurality of piston heads are line up within a distance to separate said empty space of said body into a plurality of separate cavities; and
each of said plurality of separate cavities comprises a drain wherein said drain is used to balance the pressure inside said plurality of separate cavities and for fluid flow, and each of said drain is located at the surface of said second sidewall of said body.

2. The syringe with separate cavities of claim 1 wherein said drain further comprises a conduit, the depth of said conduit is less than the difference between said diameter and said first caliber, the length of said conduit is slightly longer than the thickness of each said plurality of piston heads and is slightly less than the total thickness of said plurality of piston heads.

3. The syringe with separate cavities of claim 1 wherein said drain further comprises a conduit to communicate said each separate cavity, both ends of said conduit are opened at the surface of said second sidewall, said first sidewall is penetrated by said conduit, the length between both ends of said conduit is slightly longer than the thickness of each said plurality of piston heads and is slightly less than the total thickness of said plurality of piston heads.

4. The syringe with separate cavities of claim 1 wherein at least two fastening elements are located at the corresponding positions on the sidewall of said straight elongated stem such that said straight elongated stem can be fixed to said open end of said body in order to prevent said plurality piston heads dragging by the negative pressure inside said plurality of separate cavities, each said fastening element further comprises an elastic element and a flanker wings, a hole is placed at the sidewall of said straight elongated stem, said flanker wing of said fastening element can be swung in and out of said hole freely.

5. A syringe with separate cavities, comprising:
an empty body wherein a first end of said body is an open end with a first caliber, a second end of said body is stretched as a nozzle with a second caliber which is smaller than said first caliber, the empty space inside said body is bounded by a first sidewall with a diameter and a second sidewall with said first caliber, at least a leading guide is located at the surface of the second sidewall, said leading guide extends a first distance;
an plunger assembly which can be plug-in said empty body wherein said plunger assembly further comprises a straight elongated stem and a first piston head and a plurality of second piston heads with said first caliber, a first end of said straight elongated stem is a handle, a second end of said straight elongated stem is connected to said first piston head; and
at least a flute on the surface of all said first piston head and said plurality of second piston heads, said plurality of second piston heads are line up within a second distance from said first piston head to separate empty space of said body into a plurality of separate cavities.

6. The syringe with separate cavities of claim 5 wherein said leading guide comprises a plurality of protrusion bars, said plurality of protrusion bars are line up within said distance, said first distance is slightly longer than the total thickness of said plurality of second piston heads and is slightly less than the total thickness of a first piston head and one of said plurality of second piston heads.

7. The syringe with separate cavities of claim 5 wherein said second distance is the length of each said plurality of separate cavities.

8. The syringe with separate cavities of claim 5 wherein at least two fastening elements are located at the corresponding positions on the sidewall of said straight elongated stem such that said straight elongated stem can be fixed to said open end of said body in order to prevent said plurality piston heads dragging by the negative pressure inside said plurality of separate cavities, each said fastening element further comprises an elastic element and a flanker wings, a hole is placed at the sidewall of said straight elongated stem, said flanker wing of said fastening element can be swung in and out of said hole freely.

9. A syringe with separate cavities, comprising:
an empty body wherein a first end of said body is an open end with a first caliber, a second end of said body is stretched as a nozzle with a second caliber which is smaller than said first caliber, the empty space inside said body is bounded by a first sidewall with a diameter and a second sidewall with said first caliber, at least a protrusion is located at the surface of said second sidewall;
an plunger assembly which can be plug-in said empty body wherein said plunger assembly further comprises a straight elongated stem, a first piston head and a second piston head with said first caliber, a first end of said straight elongated stem is a handle, a second end of said straight elongated stem is connected to said first piston head; and
said second piston head is line up within a distance from said first piston head to separate empty space of said body into a first separate cavity and a second separate cavity which is connected to said nozzle.

10. The syringe with separate cavities of claim 9 wherein at least two fastening elements are located at the corresponding positions on the sidewall of said straight elongated stem such that said straight elongated stem can be fixed to said open end of said body in order to prevent said plurality piston heads dragging by the negative pressure inside said plurality of separate cavities, each said fastening element further comprises an elastic element and a flanker wings, a hole is placed at the sidewall of said straight elongated stem, said flanker wing of said fastening element can be swung in and out of said hole freely, said second separate cavity, connecting to said nozzle, is some part of empty space of said body which comprises said protrusion.
